# EUROPEAN PATENT SPECIFICATION

(11) **EP 1 013 304 B1**
(45) Date of publication and mention of the grant of the patent: **31.07.2002**
(21) Application number: 99850202.5
(22) Date of filing: 16.12.1999
(51) Int. Cl.: A61N 1/05

(54) **A device for guiding and positioning leads**
Vorrichtung zum Führen und zum Positionieren von Leitungen
Dispositif permettant de guider et de positionner des conducteurs

(30) Priority: 18.12.1998 SE 9804433
(43) Date of publication of application: 28.06.2000
(73) Proprietor: St. Jude Medical AB, 175 84 Järfälla (SE)
(72) Inventor: Hirschberg, Jakub, 183 44 Täby (SE); Strandberg, Hans, 172 65 Sundbyberg (SE); Lindegren, Ulf, 112 65 Stockholm (SE)

(56) References cited:
- US-A- 5 003 990
- US-A- 5 046 497
- US-A- 5 139 033
- US-A- 5 902 331

## Description

### Field of the invention

The present invention relates generally to a device for transvenous guidance and positioning of a distal end portion of an elongate element into a body cavity. The invention also relates to a device for implanting an electrode cable into a human heart. The devices comprise a guide wire unit having an outer flexible tubular sleeve and an inner flexible stylet, said tubular sleeve and stylet being coaxially arranged for mutual telescopical movements.

Thus, although the device of the present invention is most preferably used in implanting an electrode cable into the heart for intracardiac stimulation and/or sensing of heart signals, i.e. the device being adapted to be disconnected from the electrode cable after fixation thereof in the heart, the device may as well be used for other diagnostic and therapy purposes, such as for introducing and positioning of an optical or electric lead in the heart for e.g. the ablation of nerve paths, (for example, by laser burning), visual inspection etc., in which case the device is adapted to be withdrawn from the site of activity together with the elongate element.

### Description of prior art

Conventional medical electrode cables intended for implantation in human body cavities, in particular for intracardiac stimulation and/or sensing of heart signals, normally have a central channel or lumen defined by one or more coaxially coiled, insulated conductors. In order to advance the electrode cable through e.g. a vein to the heart while stiffening the flexible cable, a guide wire or a so-called stylet is inserted into the central lumen of the cable. By e.g. simultaneously maintaining the position of the distal end of the stylet rela-tive to the distal tip of the electrode cable and by retracting the tubular sleeve from a pre-bent end portion of the internal stylet, the deflection of the distal end of the cable may be controlled so that the tip is guided towards the desired anchoring site in a ventricle or atrium of the heart.

Due to the helical coiling of one or more conductors around the central lumen of the electrode cable, the diameter of the latter is relatively large (about 2 mm) in relation to the diameter of the individual conductors.

Recently, electrode cables have been developed which are thinner and more pliant than known unipolar and bipolar electrode cables and which consist of individual straight or twisted insulated wires occupying a space which would normally have served as a central stylet channel; see e.g. US-A-5 713 943. Such thin electrode cables, or other electrode cables which lack a central channel, need an external guide wire or stylet unit for the implantation of the cable. To this end, the electrode cable disclosed in US-A-5 713 943 has an integral exterior tube which forms a channel for a stylet and is made of a biode-gradable polymer material. The exterior tube makes the electrode cable relatively wide during the implantation stage and a certain period of time thereafter, before it degrades or dissolves.

US-A-5 003 990 and 5 304 218 disclose external guide wire arrangements for implantation of electrode cables or catheters into human hearts, wherein the cable or catheter is pushed and guided along a pre-inserted guide wire by means of a slide member. After the implantation of the electrode cable or catheter, the guide wire may be retracted from the human body while leaving the electrode/catheter at its implantation site.

### Summary of the invention

In view of the prior art discussed above, according to one aspect of the present invention, an improved device for transvenous guidance and positioning of a distal end portion of an electrode cable is provided, in which the elongate element may be pulled and simultaneously introduced by an external guide wire unit all the way to the site for diagnostics and therapy in the cavity of the human body and properly guided to that site.

Furthermore, it is an object of the present invention to provide an improved electrode cable implanting device, in which the electrode cable may be pulled and simultaneously introduced by an external guide wire unit all the way to the implantation site in the cavity of the human body, and then, after fixation of the distal tip of the electrode cable, released therefrom for retraction from the human body. To this end, the device as defined in the preamble of claim 1 in accordance with the present invention is designed in accordance with the characterizing portion of claim 1.

By using such an external guide wire unit which is adapted to be connected to the distal end portion of the elongate element prior to the insertion thereof and to pull the element to its operating site within a cavity of the human body, in a common, simultaneous insertion with the guide wire unit, it will become possible to insert and guide thinner elongate elements which need not have a central lumen for an internal guide wire unit.

Alternatively, electrode cables of normal diameter size may include more conductors in the central area which otherwise would have been occupied by the internal guide wire. After the implantation of the electrode cable, the external guide wire unit of the present invention in one embodiment may be actuated so as to be released from the distal end portion of the cable and then retracted from the human body.

Details and features of practical and preferred embodiments of the present invention are defined in the accompanying dependent claims and will be further explained in the following detailed description with references to the accompanying drawings.

### Brief description of the drawings

Fig. 1 is an enlarged side elevational view of a distal end portion of an electrode cable to which is temporarily attached a retainer member of an external guide wire unit of the present invention;
Fig. 2 is a side elevational view similar to that of Fig. 1 but shows a stage in which the retainer member of the guide wire unit is being released from the electrode cable after the implantation thereof;
Fig. 3 is a transverse cross-section taken along the line III-III in Fig. 1;
Figs. 4a-4c are schematic side elevational views of three different operative positions of the guide wire device connected to a manipulation handle;
Fig. 5a is a side elevational view of an alternative embodiment of a device for transvenous guidance and positioning of an electrode cable, in accordance with the present invention, during attachment of the distal end of the cable to the distal end of the sleeve of a guide wire unit by means of a loop-shaped retainer;
Fig. 5b is a view similar to Fig.5a illustrating the distal end of the cable attached to the sleeve; and
Fig. 5c shows the step of detaching the cable from the guide wire.

### Description of a preferred embodiment

An external guide wire unit 10 of the present invention for use in implanting a medical electrode cable 12 in a cavity of a human body, such as a ventricle or atrium of a heart, comprises an outer elongate, flexible, tubular sleeve 14 and an inner flexible stylet 16. The sleeve 14 and the stylet 16 are movable in a telescopic manner relative to each other. Preferably, the cross-section of the stylet 16 is non-circular in order to prevent mutual rotation between the stylet and the sleeve, such as disclosed in US-A-5 728 148.

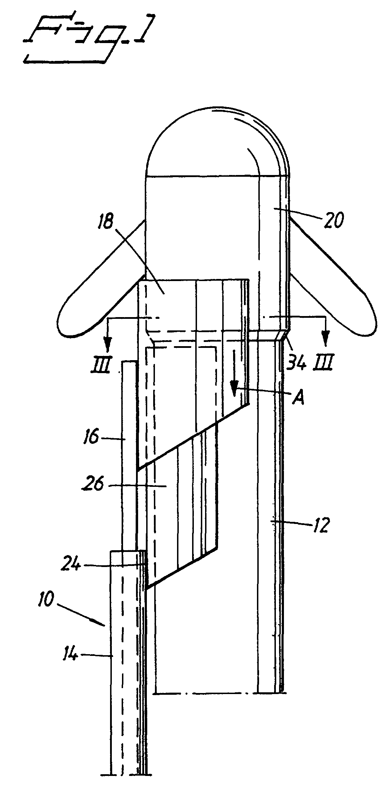

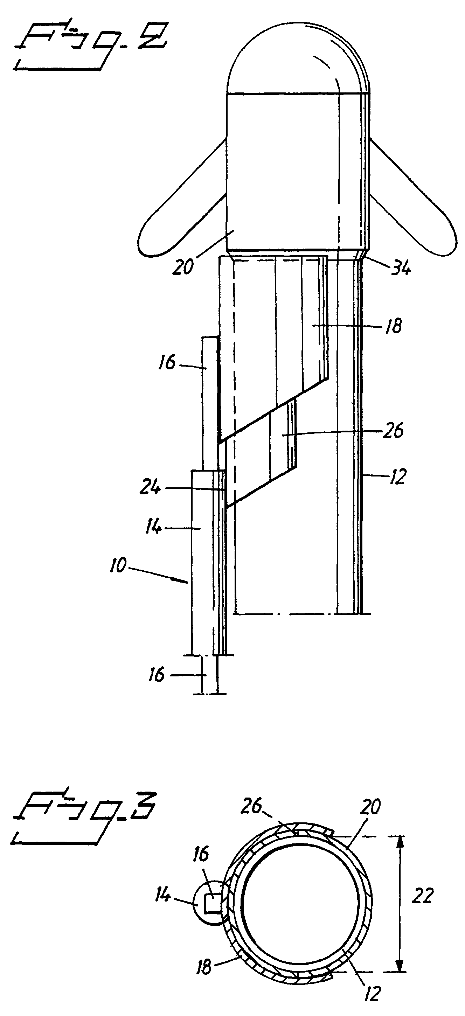

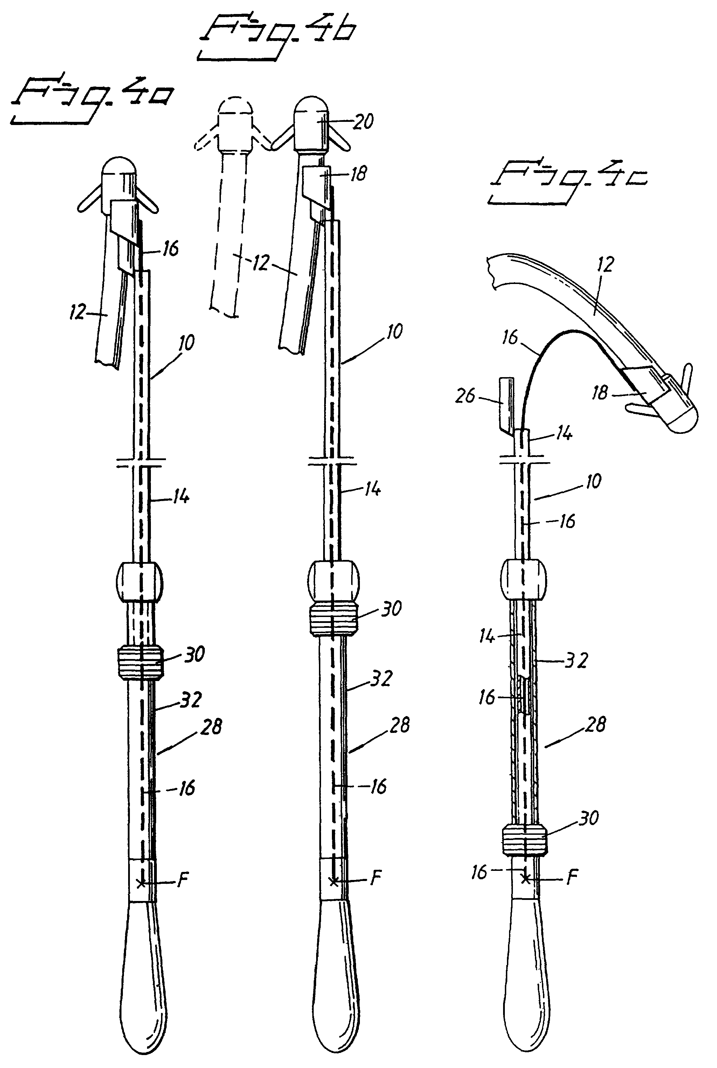

An electrode retainer 18 is attached to a distal end portion of the stylet 16 and has the shape of a resilient tubular clamp having a substantially C-formed cross-section (Fig. 3) so as to be able to circumscribe more than 180° but less than 360° of the circumference of an enlarged distal head portion 20 of the electrode cable 12. Thus, the tubular clamp 18 defines a longitudinal slot 22 having a width which is less than the diameter of the head portion 20.

To the distal end portion 24 of the sleeve 14 is attached a slide member 26 having a substantially semicircular cross-section of less diameter than the clamp 18, thereby enabling the slide member 26 to be displaced along the outer surface of the electrode cable 12 and in a narrow space between the latter and the clamp 18, i.e. to be displaced telescopically inside the clamp 18. The slide member 26 also preferably has a substantially C-shaped cross-section but is designed to circumscribe less than 180° of the circumference of the electrode cable.

As a preparation, before inserting the electrode cable 12 into a vein for the implantation of the cable in the heart, the clamp 18 is radially snap-fitted (or pushed) onto the enlarged head portion 20 of the cable while allowing the tubular wall portions of the clamp 18 to flex outwardly and then return to a biased gripping position (Fig. 1) to securely hold the electrode head portion 20 during the insertion and implantation phases of the cable.

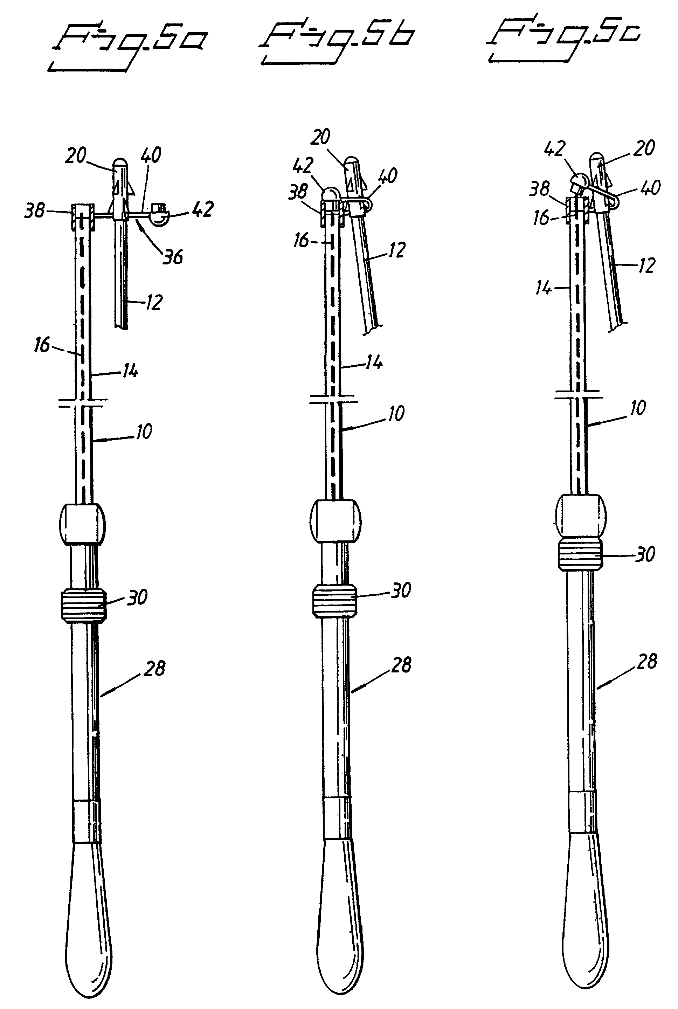

The proximal end of the stylet 16 is preferably fixated at F in a control or manipulation handle 28 (Figs. 4a-4c), while the rear or proximal end of the tubular sleeve 14 of the guide wire unit 10 is secured to a slide ring 30 which is displaceable by the fingers of the operator on a shaft portion 32 of the handle 28. When mounting the clamp 18 onto the enlarged head portion 20, the slide ring 30 takes the position shown in Fig. 4a.

The electrode cable 12 is advanced through the vein on its way to the heart by pushing the guide wire unit 10, thereby entraining or pulling the cable 12 through the vein. The guidance of the distal end of the electrode cable through the vein and the respective cavity of the heart may be controlled by displacing the outer sleeve 14 relative to the stylet 16 and/or rotating the guide wire unit together with the electrode cable 12. For example, when retracting the sleeve 14 in the direction of the arrow A in Fig. 1 by means of the slide ring 30 of the control handle outside the human body, a pre-bent portion of the stylet 16 within the sleeve 14 will be exposed and cause the distal end portion 20 of the elec-trode cable 12 to be deflected or curved in a desired manner (see Fig. 4c). This is espe-cially the case when locating a suitable position for the electrode tip in the ventricle or atrium of the heart.

After fixation of the electrode tip at a suitable site for pacing and/or sensing, the retainer (clamp) 18 may be released or disconnected from the distal electrode head portion 20 by pushing the sleeve 14 and the slide member 26 forwardly to a position (Fig 4a) in which the slide member 26 comes to engagement with a radial shoulder 34 formed at a diameter transition between the distal head portion 20 and the electrode cable 12. Alternatively, end stops such as lugs or protrusions (not shown) may be provided in lieu of the radial shoulder 34 to limit the forward displacement of the slide member 26. When the position shown in Fig. 2 and Fig. 4a has been reached, a further forwardly directed force applied to the slide ring 30 and thus the sleeve 14 and the slide member 26 will exceed the fric-tional engagement between the retainer 18 and the head portion 20 and thus result in a rearwardly directed displacement of the stylet 16 to release the retainer 18 from the elec-trode head portion 20 (see the position in Fig. 4b). Then, the guide wire unit 10 may be retracted by pulling it out from the heart and the respective veins.

If desired, this retraction of the guide wire unit 10 may be guided by the remaining electrode cable 12 by selecting a width dimension of the slot 22 that is smaller than the diameter of the electrode cable 12. This will prevent a separation of the retainer 18 from the electrode cable 12 during retraction. The rear or tail end of the retainer 18 and the slide member 26 are preferably tapered or rounded so as to prevent them from damaging the veins or other obstacles during the retraction of the guide wire unit 10.

In the embodiment shown in Figs. 1-3, however, the width of the slot 22 is somewhat larger than the diameter of the electrode cable 12. This will enable the guide wire unit 10 to be fully disconnected from the electrode cable 12 after the release of the retainer 18 from the electrode head portion 20, thereby unnecessitating a separation of the guide wire unit 10 from the electrode cable 12 after the withdrawal thereof from the human body. Also, the risk of undue engagement between the electrode cable 12 and the guide wire unit 10 during the retraction phase of the latter will be reduced.

Figs. 5a-c show an alternative embodiment of the device according to the present invention for transvenous guiding and positioning of an electrode cable 12 by means of a guide wire unit 10 located externally of the electrode cable 12. Attached to the distal end of the sleeve 14 is a cable tip retainer 36 comprising a socket member 38 fixated to said distal end, a loop-forming thread or string 40, and a cap member 42 adapted to fit into the socket member 38, as shown in Fig. 5b, such that the string 40 forms a loop around the tip end of the electrode cable to thereby hold the same during implantation thereof into e.g. a human heart. After completion of the implantation, the stylet 16 is displaced forwardly by pushing the slide ring 30 of the manipulation handle 28 forwardly, whereby the distal end of the stylet 16 will eject the cap member 42 from the socket member 38 so that the string 40 will release the electrode tip from the guide wire unit 10, as shown in Fig. 5c. Then, the guide wire unit 10 may be withdrawn from the implantation site leaving the electrode at site. Thus, the embodiment of Figs. 5a-c is likewise adapted to release the electrode tip by a mutual telescopical movement between the stylet 16 and the sleeve 14.

Preferably, the distal end portion of the sleeve 14 of the guide wire unit 10 for manipulating the tip retainer 36 is pre-bent and adapted, during withdrawal of the stylet tip 16 from the distal end portion of the sleeve 14, to deflect the distal end portion of the electrode cable 12 so as to facilitate insertion thereof during implantation.

## Claims

1. A device for transvenous guidance and positioning of a distal end portion of an electrode cable (12) into a body cavity, such as a human heart, comprising a guide wire unit (10) being adapted to be arranged externally of said electrode cable and having an outer flexible tubular sleeve (14) and an inner flexible stylet (16), said tubular sleeve (14) and stylet (16) being coaxially arranged for mutual telescopical movements, **characterized in that** a distal end portion of the stylet (16) or of the tubular sleeve (14) is designed to be releasably attached to a distal end portion (20) of the electrode cable (12) and adapted to be detached therefrom by a mutual telescopical movement between the stylet (16) and the sleeve (14).

2. A device according to claim 1, **characterized in that** said distal end portion of the stylet (16) is adapted to be releasably attached to said distal end portion (20) of the electrode cable (12), and that a distal end portion (24) of the sleeve (14) is adapted, during a phase of mutual movement between the sleeve and the stylet, to detach the stylet (16) from the electrode cable (12).

3. The device according to Claim 2, **characterized in that** the distal end portion of the stylet (16) is provided with a retainer means (18) adapted to be releasably attached to the outer perimeter of the distal end portion (20) of the electrode cable (12), and that the distal end portion (24) of the sleeve (14) is provided with a slide member (26).

4. The device according to Claim 3, **characterized in that** the retainer means has the shape of a resilient clamp (18), said clamp (18) having a tubular configuration with a substantially C-shaped cross-section with a longitudinal slot (22) and being adapted to grip an enlarged portion of the distal end portion (20) of the electrode cable (12).

5. The device according to Claim 4 **characterized in that** the slot (22) has a width which exceeds the outer diameter of the electrode cable (12) to be implanted.

6. The device according to Claim 5, **characterized in that** the slot (22) has a width which is less than the outer diameter of the electrode cable to be implanted.

7. The device according to anyone of Claims 3 - 6, **characterized in that** the slide member (26) is arranged to engage a stop means at the distal end portion (20) of the electrode cable, to thereby generate a rearwardly directed retraction of the clamp from the enlarged portion of said end portion during said phase of mutual movement between the sleeve (14) and the stylet (16).

8. The device according to Claim 7, **characterized in that** the slide member (26) is adapted to move telescopically inside the clamp.

9. The device according to Claim 7 or 8, **characterized in that** the slide member (26) has a maximal extension of 180° in the circumferential direction.

10. The device according to Claim 9, **characterized in that** the slide member (26) has a radius of curvature substantially corresponding to the radius to the electrode cable (12) to be implanted.

11. The device according to any one of claims 2-10, **characterized in that** a distal portion of the stylet (16) is pre-bent and adapted, during a phase of exposure from the sleeve, to deflect the distal end portion of the electrode cable (12).

12. The device according to claim 1, **characterized in that** the distal end portion of the sleeve (14) is adapted to be releasably attached to the distal end portion (20) of the electrode cable (12), and that a distal end portion of the stylet (16) is adapted, during a phase of mutual movement between the sleeve and the stylet, to detach the sleeve (14) from the electrode cable (12).

13. The device according to claim 12, **characterized in that** the distal end portion of the sleeve (14) is provided with a retainer means (36) adapted to be releasably attached to the distal end portion (20) of the electrode cable (12).

14. The device according to claim 13, **characterized in that** the retainer means comprises a loop-forming thread element (40) having one end (38) fixedly attached to the distal end portion of the sleeve (14) and another end (42) capable of being releasably attached to said one end (38) or to the distal end portion of the sleeve (14).

15. The device according to any one of claims 12-14, **characterized in that** a distal portion of the sleeve (14) is pre-bent and adapted, during withdrawal of the stylet tip from the distal end portion of the sleeve, to deflect the distal end portion of the electrode cable (12).

## Patentansprüche

1. Vorrichtung zum transvenösen Führen und Positionieren des distalen Endabschnittes eines Elektrodenkabels (12) in einem Körperhohlraum, wie einem menschlichen Herzen, enthaltend eine Führungsdrahteinheit (10), die ausgelegt ist, außerhalb des Elektrodenkabels angeordnet zu werden und eine äußere flexible rohrförmige Hülse (14) und einen inneren biegsamen Mandrin (16) aufweist, wobei die rohrförmige Hülse (14) und der Mandrin (16) koaxial zueinander angeordnet sind, für gegenseitige teleskopische Bewegungen, **dadurch gekennzeichnet, daß** ein distaler Endabschnitt des Mandrins (16) oder der rohrförmigen Hülse (14) so ausgebildet ist, daß er lösbar an einem distalen Endabschnitt (20) des Elektrodenkabels (12) angebracht und geeignet ist, von diesem gelöst zu werden, durch eine gegenseitige teleskopische Bewegung zwischen dem Mandrin (16) und der Hülse (14).

2. Vorrichtung nach Anspruch 1, **dadurch gekennzeichnet, daß** der distale Endabschnitt des Mandrins (16) ausgelegt ist, lösbar an dem distalen Endabschnitt (20) des Elektrodenkabels (12) angebracht zu werden und daß ein distaler Endabschnitt (24) der Hülse (14) ausgelegt ist, während einer Phase einer gegenseitigen Bewegung zwischen der Hülse und dem Mandrin, den Mandrin (16) von dem Elektrodenkabel (12) zu lösen.

3. Vorrichtung nach Anspruch 2, **dadurch gekennzeichnet, daß** der distale Endabschnitt des Mandrins (16) mit einer Haltevorrichtung (18) versehen ist, die ausgelegt ist, lösbar am äußeren Umfang des distalen Endabschnittes (20) des Elektrodenkabels (12) angebracht zu werden und daß der distale Endabschnitt (24) der Hülse (14) mit einem Gleitelement (26) versehen ist.

4. Vorrichtung nach Anspruch 3, **dadurch gekennzeichnet, daß** die Haltevorrichtung die Form einer federnden Klemme (18) aufweist, die Klemme (18) eine rohrförmige Gestalt mit einem im wesentlichen (C-förmigen) Querschnitt mit einem Längsschlitz (22) besitzt und geeignet ist, einen vergrößerten Abschnitt des distalen Endabschnittes (20) des Elektrodenkabels (12) zu greifen.

5. Vorrichtung nach Anspruch 4, **dadurch gekennzeichnet, daß** der Schlitz (22) eine Breite aufweist, die den äußeren Durchmesser des zu implantierenden Elektrodenkabels (12) überschreitet.

6. Vorrichtung nach Anspruch 5, **dadurch gekennzeichnet, daß** der Schlitz (22) eine Breite aufweist, die kleiner ist als der Außendurchmesser des zu implantierenden Elektrodenkabels.

7. Vorrichtung nach einem der Ansprüche 3 bis 6, **dadurch gekennzeichnet, daß** das Gleitelement (26) so angeordnet ist, daß es mit einem Anschlag am distalen Endabschnitt (20) des Elektrodenkabels in Eingriff gebracht werden kann, um hierdurch ein nach rückwärts gerichtetes Zurückziehen der Klemme aus dem vergrößerten Abschnitt des Endabschnittes, während der Phase der gegenseitigen Bewegung zwischen der Hülse (14) und dem Mandrin (16) zu erzeugen.

8. Vorrichtung nach Anspruch 7, **dadurch gekennzeichnet, daß** das Gleitelement (26) ausgelegt ist, sich teleskopisch innerhalb der Klemme zu bewegen.

9. Vorrichtung nach Anspruch 7 oder 8, **dadurch gekennzeichnet, daß** das Gleitelement (26) in Umfangsrichtung eine maximale Erstreckung von 180° aufweist.

10. Vorrichtung nach Anspruch 9, **dadurch gekennzeichnet, daß** das Gleitelement (26) einen Krümmungsradius aufweist, der im wesentlichen dem Radius des zu implantierenden Elektrodenkabels (12) entspricht.

11. Vorrichtung nach einem der Ansprüche 2 bis 10, **dadurch gekennzeichnet, daß** ein distaler Abschnitt des Mandrins (16) vorgebogen und ausgelegt ist, in einer Phase der Freilegung aus der Hülse den distalen Endabschnitt des Elektrodenkabels (12) zu krümmen.

12. Vorrichtung nach Anspruch 1, **dadurch gekennzeichnet, daß** der distale Endabschnitt der Hülse (14) ausgelegt ist, lösbar am distalen Endabschnitt (20) des Elektrodenkabels (12) angebracht zu werden und daß ein distaler Endabschnitt des Mandrins (16) ausgelegt ist, während einer Phase einer gegenseitigen Bewegung zwischen der Hülse und dem Mandrin, die Hülse (14) von dem Elektrodenkabel (12) zu lösen.

13. Vorrichtung nach Anspruch 12, **dadurch gekennzeichnet, daß** der distale Endabschnitt der Hülse (14) mit einer Haltevorrichtung (36) versehen ist, die ausgelegt ist, lösbar am distalen Endabschnitt (20) des Elektrodenkabels (12) angebracht zu werden.

14. Vorrichtung nach Anspruch 13, **dadurch gekennzeichnet, daß** die Haltevorrichtung ein schleifenförmiges Fadenelement (40) enthält, dessen eines Ende (38) fest am distalen Endabschnitt der Hülse (14) angebracht ist und dessen anderes Ende (42) in der Lage ist, lösbar an dem einen Ende (38) oder dem distalen Endabschnitt der Hülse (14) angebracht zu werden.

15. Vorrichtung nach einem der Ansprüche 12 bis 14, **dadurch gekennzeichnet, daß** ein distaler Abschnitt der Hülse (14) vorgebogen und ausgelegt ist, beim Zurückziehen der Mandrinspitze aus dem distalen Endabschnitt der Hülse den distalen Endabschnitt des Elektrodenkabels (12) zu krümmen.

## Revendications

1. Dispositif pour le guidage et le positionnement transveineux d'une partie d'extrémité distale d'un câble (12) d'électrode dans une cavité de corps, tel qu'un coeur humain, comportant une unité (10) formant fil de guidage qui est conçue pour être disposée à l'extérieur du câble d'électrode et ayant un manchon (14) extérieur tubulaire souple et un stylet (16) intérieur souple, le manchon (14) tubulaire et le stylet (16) étant disposés coaxialement pour des mouvements télescopiques mutuels, **caractérisé en ce qu'**une partie d'extrémité distale du stylet (16) ou du manchon (14) tubulaire est conçue pour être fixée de manière amovible à une partie (20) d'extrémité distale du câble (12) d'électrode et elle est conçue pour être détachée de celui-ci par un mouvement télescopique mutuel entre le stylet (16) et le manchon (14).

2. Dispositif suivant la revendication 1, **caractérisé en ce que** la partie d'extrémité distale du stylet (16) est conçue pour être fixée de manière amovible à la partie (20) d'extrémité distale du câble (12) d'électrode, et **en ce qu'**une partie (24) d'extrémité distale du manchon (14) est conçue, pendant une phase de déplacement mutuel entre le manchon et le stylet, pour détacher le stylet (16) du câble (12) d'électrode.

3. Dispositif suivant la revendication 2, **caractérisé en ce que** la partie d'extrémité distale du stylet (16) est munie de moyens (18) de retenue conçus pour être fixés de manière amovible à la périphérie extérieure de la partie (20) d'extrémité distale du câble (12) d'électrode, et **en ce que** la partie (24) d'extrémité distale du manchon (14) est munie d'un élément (26) coulissant.

4. Dispositif suivant la revendication 3, **caractérisé en ce que** les moyens de retenue ont la forme d'un clip (18) élastique, le clip (18) ayant une configuration tubulaire ayant une section transversale sensiblement en forme de C, avec une fente (22) longitudinale, et étant conçu pour serrer une partie agrandie de la partie (20) d'extrémité distale du câble (12) d'électrode.

5. Dispositif suivant la revendication 4, **caractérisé en ce que** la fente (22) a une largeur qui dépasse le diamètre extérieur du câble (12) d'électrode à implanter.

6. Dispositif suivant la revendication 5, **caractérisé en ce que** la fente (22) a une largeur qui est moindre que le diamètre extérieur du câble d'électrode à implanter.

7. Dispositif suivant l'une quelconque des revendications 3 à 6, **caractérisé en ce que** l'élément (26) coulissant est disposé pour coopérer avec des moyens de butée à la partie (20) d'extrémité distale du câble d'électrode, pour ainsi produire un retrait dirigé vers l'arrière du clip de la partie agrandie de la partie d'extrémité pendant la phase de mouvement mutuel entre le manchon (14) et le stylet (16).

8. Dispositif suivant la revendication 7, **caractérisé en ce que** l'élément (26) coulissant est conçu pour se déplacer télescopiquement à l'intérieur du clip.

9. Dispositif suivant la revendication 7 ou 8, **caractérisé en ce que** l'élément (26) coulissant a une extension maximale de 180° dans la direction circonférentielle.

10. Dispositif suivant la revendication 9, **caractérisé en ce que** l'élément (26) coulissant a un rayon de courbure qui correspond sensiblement au rayon du câble (12) d'électrode à implanter.

11. Dispositif suivant l'une quelconque des revendications 2 à 10, **caractérisé en ce qu'**une partie distale du stylet (16) est précourbée et conçue, pendant une phase d'exposition du manchon, pour dévier la partie d'extrémité distale du câble (12) d'électrode.

12. Dispositif suivant la revendication 1, **caractérisé en ce que** la partie d'extrémité distale du manchon (14) est conçue pour être fixée de manière amovible à la partie (20) d'extrémité distale du câble (12) d'électrode, et **en ce qu'**une partie d'extrémité distale du stylet (16) est conçue, pendant une phase de mouvement mutuel entre le manchon et le stylet, pour détacher le manchon (14) du câble (12) d'électrode.

13. Dispositif suivant la revendication 12, **caractérisé en ce que** la partie d'extrémité distale du manchon (14) est munie de moyens (36) de retenue conçus pour être fixés de manière amovible à la partie (20) d'extrémité distale du câble (12) d'électrode.

14. Dispositif suivant la revendication 13, **caractérisé en ce que** les moyens de retenue comportent un élément (40) chaîne formant boucle ayant une extrémité (38) fixée de manière fixe à la partie d'extrémité distale du manchon (14) et une autre extrémité (42) capable d'être fixée de manière amovible à ladite une extrémité (38) ou à la partie d'extrémité distale du manchon (14).

15. Dispositif suivant l'une quelconque des revendications 12 à 14, **caractérisé en ce qu'**une partie distale du manchon (14) est précourbée et est conçue, pendant le retrait de la pointe de stylet de la partie d'extrémité distale du manchon, pour dévier la partie d'extrémité distale du câble (12) d'électrode.
